Europäisches Patentamt

(19) European Patent Office

Office européen des brevets

(11) Publication number: **0 234 941**
**A2**

# (12) EUROPEAN PATENT APPLICATION

(21) Application number: **87301714.9**

(22) Date of filing: **26.02.87**

(51) Int. Cl.⁴: **G 01 N 33/569**

(30) Priority: **26.02.86 US 833072**

(43) Date of publication of application:
**02.09.87 Bulletin 87/36**

(84) Designated Contracting States:
**AT BE CH DE FR GB IT LI LU NL SE**

(71) Applicant: **THE UNITED STATES OF AMERICA represented by The Secretary The United States Department of Commerce**
**5285 Port Royal Road**
**Springfield Virginia 22161 (US)**

(72) Inventor: **Gallo, Robert Charles**
**8513 Thornden Terrace**
**Bethesda Maryland 20834 (US)**

**Saxinger, William Carl**
**6814 Renita Lane**
**Bethesda Maryland 20817 (US)**

(74) Representative: **Jump, Timothy John Simon**
**F.J. Cleveland and Company 40-43 Chancery Lane**
**London WC2A 1JQ (GB)**

(54) Competitive elisa for the detection of antibodies.

(57) A competitive enzyme-linked immunosorbent assay (ELISA) for the detection of antibodies is disclosed. This ELISA technique is more sensitive, more specific, and more accurate than known ELISA techniques. The competitive ELISA of this invention is particularly suited to the detection of human T-cell leukemia-lymphoma virus type III (HTLV-III). An improvement of the ELISA technique represented by the present invention lies in the use of a reaction buffer which suppresses non-specific adsorptive reactions between the test substrate and the test antigen. Furthermore, the ELISA technique is improved by removing contaminants from the target atnigen preparation.

**0 234 941**

**Description**

## COMPETITIVE ELISA FOR THE DETECTION OF ANTIBODIES

The present invention is a specific and sensitive method of detecting antibodies in test sera. The method, competitive enzyme-linked immunosorbent assay (ELISA), was developed, in particular, for the detection of the human T-cell leukemia-lymphoma virus type III (HTLV-III), the putative causative agent of acquired immune deficiency syndrome (AIDS). This method, however, is generally applicable to the HTLV family of viruses, as well as other antigens and their corresponding antibodies.

Many immunoassay techniques exist for the immunosurveilance of antigens and antibodies. The indirect ELISA assay and the "Western" electroblotting assays are among the most sensitive. However, one of the drawbacks of these procedures is the background interference caused by nonimmune immunoglobulin and other serum factors present in all normal sera. This background, by its tendency to overlap and obscure low levels of specific reactive immunoglobulin, decreases the sensitivity of the assay and creates the need for supplementary confirmation tests in order to demonstrate true positivity. One such confirming test used extensively is the "Western" blotting procedure which, although sensitive, more specific, and informative for distinct viral proteins, is highly labor intensive, difficult to interpret, and is qualitative in nature (rather than quantitative).

The competitive ELISA immunoassay of the present invention greatly increases the sensitivity, specificity, and convenience of obtaining serologic data and detecting HTLV-III antibody. The method increases sensitivity of specific antibody measurements by decreasing the background of normal immunoglobulin. The method increases specificity by the use of heterologous antiserum prepared against a virus, thus competitively blocking human specific antibodies.

According to the present invention, a quantity of human lymphocytes is utilized as a portion of the buffer in order to reduce nonspecific test serum reactions with the test antigen and substrate. Furthermore, the target antigen is treated with a guanidine salt, preferably guanidine hydrochloride, in order to increase sensitivity and specificity of the ELISA test.

ELISA assays have proven effective but are limited by reactions between the test substrate and the animal anti-sera. It is a function of the present invention to provide a method which decreases and diminishes such non-specific reactions. This is accomplished, in part, by increased antigen purity--treating the target antigen with a guanidine salt, such as guanidine hydrochloride, or a similar salt where the counter ion is another halogen, such as bromine or iodine, or a thiocyanate or nitrate. These salts are utilized at a concentration which achieves protein denaturation (approximately 4-8 molar solution).

The present invention is particularly suited to the detection of HTLV-III antibodies. Since AIDS and HTLV-III are known to be transmitted by blood products, a method for detecting contamination with HTLV-III in such blood products is needed to guarantee the safety of recipients. Such a method should be as sensitive and specific as possible, and should also be easily performed in a routine environment. Tests for viral markers such as reverse transcriptase, viral antigens, and nucleic acid sequences in fresh or cultured blood cells are slow and pressently not suitble for large-scale screening. On the other hand, serological tests for viral antibodies or antigens are well suited for this purpose, since HTLV-III infected individuals are usually seropositive for antibodies to one or more HTLV-III proteins.

Statement of Deposit

HTLV-III was deposited in the American Type Culture Collection (ATCC), 12301 Parklawn Drive, Rockville, MD 20852, U.S.A., for a term of at least thirty years or five years after the last sample request, whichever is longer. These deposits were as follows:

ATCC No. CRL 8602 August 15, 1984
ATCC Nos. 40125, 40126, 40127 July 30, 1984
ATCC No. CRL 8543 April 19, 1984

Enzyme-linked immunosorbent assays (ELISA), in general, are performed by binding a reference reagent (antigen) to a solid phase support. Test sera, mixed with a labeled reagent, is then reacted with the bound reference reagent. The reagents are then subjected to a series of dilution, incubation, and washing steps in order to separate bound and free reagents. The process concludes with a detection step, compatible with the type of label used, designed to indirectly measure the amount of antibody (or antigen) in the test sera.

The older, conventional ELISA system occasionally produces false-positve results. Generally, antigen or virus fragments are bound to a solid support. The bound fragments are then incubated with heteroantiserum which binds to the bound fragments. Test sera is then added to the bound fragments and tested for absorbance. Suppression of absorbance by greater than 50 percent (relative to normal human serum) is considered a positive test result. The following description, using HTLV-III as the antigen, more specifically describes the present invention.

HTLV-III antigents are purified by rate-zonal ultracentrifugation, disrupted, clarified by ultracentrifugation, and coated in the wells of microtiter plates. Test sera is tested and confirmed by a confirmatory neutralization screening which includes an additional 2-hour incubation period before the test sample is incubated with the antigen-coated wells. During this extra 2 hours of incubation, the wells are exposed to unlabeled sheep antibody (heteroantiserum) to HTLV-III which reacts with and saturates HTLV antigen sites on the well, thus preventing the test serum from attaching to the well in the subsequent step. As a control in the test, adjacent wells are exposed to normal sheep serum during the additional incubation period. The preferred dilution of sheep antiserum is 1:2 at a titer of 10,000 or more. Sheep antiserum showing reactivity with proteins from phytohemagglutinin (PHA) stimulated human lymphocyte preparations (or uninfected host cells, e.g. H9) coated on microtiter plate wells is absorbed with PHA lymphocyte preparations (or uninfected cells) until the reactivity is removed. Preferably, the sheep antiserum used in this process may be absorbed with one volume of cell equivalents per three volumes of serum. A suppression of the absorbance by more than 50 percent in the sample exposed to the unlabeled sheep antiserum to HTLV-III, relative to a standard normal human serum, is considered a positive confirmatory result for the presence of antibody to HTLV-III.

A preferred formulation containing uninfected hoste cells or a heterologous population of normal human lymphocytes designed to block the reactivities of the "true" positive sera by the animal HTLV antisera are the following which may be termed "buffers": sheep plasma, 4/8 (optionally, 1/8 immune sheep plasma and 3/8 normal sheep plasma); a solution of 20% goat serum 20% fetal bovine serum, and phosphate buffered saline, 3/8; and lymphocyte extract, 1/8. The lymphocyte extract (a detergent extract of leucocytes) is 1 gram fresh lymphocyte suspended in 3 volumes of 5% NP40 (a non-ionic detergent, nonidet P40), 50 millimolar Tris pH 7.4 (Confer, Merck 10, Compound 9575), trasylol (FBA Pharmaceuticals, Merck 10, Compound 780), 10 millimolar magnesium chloride, and .25 molar glycerol. To produce the extract, the cells are disrupted by vortex or a loose fitting homogenizer and the nuclei are removed by low speed centrifugation (2000 RPM for 20 minutes). The remaining supernatant is the lymphocyte extract. Although any of the non-ionic detergents (i.e., a reagent which breaks down the cell wall and releases the nuclei) listed in McCutcheon's Emulsifiers & Detergents, North American Edition, 1983, may be used, the preferred non-ionic detergents are NP40 and Triton X100 (octyl phenoxy polyethoxy ethanol type compounds).

Detergent extract of leucocyte. One gram fresh (viable) lymphocyte is suspended in 3 volumes of 5% NP40, 50 millimolar Tris pH 7.4 (trimethylol aminomethane; Merck 10, Compound 9575), 1 millimolar phenyl methyl sulfonyl fluoride (PMSF, a proteinase inhibitor), and trasylol (a proteinase inhibitor), 10 millimolar magnesium chloride, and .25 molar glycerol.

An additional improvement in ELISA for the detection and diagnosis of HTLV I, II and III, is the addition to the antigen of an effective amount of a guanidine salt such as guanidine hydrochloride. Alternative guanidine salts which may be utilized contain the other halogen counter ions, such as the bromide and iodide, as well as the thiocyanate. The ultilization of GuHCl, for example, in treating the HTLV-III target antigen results in increased test sensitivity and specificity in this competitive ELISA antibody assay. The test sensitivity is 3-10 times more than the current commercial screening tests, and more specific compared with the test antigens prepared with detergent salt when dealing with a small subset of falsely reacting sera. In certain uses, the competitive ELISA antibody assay is 300 to 1000 times more sensitive than the current commercial screening tests. By following the process noted above, it is believed that certain cellular antigens are destroyed while preserving the immuno reactivity of the HTLV antigens. This efect produces a target antigen with increased specificity and senstitivity compared to existing screeing tests. A molarity range of 4-8 molar has been found expecially useful, with 6 molar guanidine salt being preferred.

Preparation of sheep anti-HTLV-III. Sheep are inoculated intradermally with 1 mg of viral proteins in complete Freund's adjuvant (incomplete Freund's is used thereafter). The protein fraction is obtained by disruption of sucrose gradient purified HTLV-III/H9 virions in nonionic detergent and 0.6 M NaCl. At the same time, 1 mg of purified virions fixed in 0.04% paraformaldehyde is administered intradermally. Booster inoculations spaced 1 month apart are administered intradermally, intramuscularly or intravenously until titers are sufficiently high. Material for applying the test is obtained a liter at a time by plasmapheresis. Plasma from nonimmune sheep is obtained by the same procedure.

While the protein fraction noted above is obtained by disruption of HTLV-III/H9 virions, practitioners in the art will understand that the invention also includes (but is not limited to) the use of purified viral protein, core antigens, and cloned subgenomic fragment derivatives. These include (but are not limited to) HTLV-I, HTLV-II, HTLV-III, segments thereof, and molecular clones BH10, HXB-2, HXB-3, BH8, BH4, and plasmids, proteins, or segments derived from these clones. Based on the present disclosure, many other modifications and ramifications will naturally suggest themselves to those skilled in the art. These alterations are within the scope of this invention.

The present invention may be practiced by use of a test kit which includes (but is not limited to) an insoluble porous surface or solid substrate, which preferably contains microtiter wells; a reagent containing an antigen or antibody to be bound to the insoluble surface or support; test sera; heteroantiserum which specifically binds to and saturates the antigen or antibody bound to the surface; and biologically suitable washing, incubating, and purifying reagents known to those skilled in the art.

The Ig component of the process is selected from any available Ig antibody such as IgA, IgD, IgE, IgG, and IgM. A preferred temperature for the process is in the cold, most preferably at or about 4°C. A range of 1-45°C may be tolerated. A preferred enzyme is horseradish peroxidase. A preferred ratio of perodixase to Ig or gamma globulin is 1:1 to 5:1.

The immunoglobulin, anti-human IgG, IgA, IgD, IgE, or IgM is preferably obtained from goats. Other animal antihuman immunoglobulin may be used, such as rabbit, hamster, chicken, rat, guinea pig, sheep, horse, or mouse. These immunoglobulins are commercially available.

The immunosorbent used in the present invention is insoluble and capable of attaching HTLV-III antigens.

This immunosorbent is present as an insoluble surface and may be agarose in bead form, carbohydrates such as dextran, cellulose, or nitrocellulose, plastics such as polystyrene, polycarbonate, polypropylene, polyamide or polyvinyl, or inorganic materials such as glass or silica gel. HTLV-III antigens may be chemically coupled to the immunosorbent or coated onto the surface of the immunosorbent, or captured by antibodies coated on the surface.

The wells (for example, of Immulon 1 microtiter plates, commercially available from Dynatech Laboratories, Inc.) may be filled with 0.1 ml of 50 mM Na HCO$_3$ (pH 9.6) containing disrupted antigen (diluted or 5 g/ml of detergent-free HTLV proteins) and incubated overnight at 4°C. The plates may be used immediately or stored at 4°C in a humidified chamber, or dried and stored dehydrated until used.

Western blotting comparative analyses were performed by a three-layer immunoperoxidase procedure.

Example 1

Specific antibody determinations in chimpanzees experimentally inoculated with AIDS plasma

Three chimpanzees (CH132, CH114, CH133) were inoculated with AIDS plasma while a fourth (CH140) was inoculated only with normal human plasma. Blood was sampled biweekly from each animal and serum was tested in the standard indirect ELISA test for HTLV-III antibodies. A P/N value of more than 5 is considered by many to indicate positive reactivity. By this criterion all four of the animals were positive for passively transferred HTLV-III antibody reactivity. CH114 and CH133 also showed active production of HTLV-III antibodies beginning six to eight weeks after the infusion of AIDS plasma. When subjected to the antibody blocking procedure of the present invention using sheep anti-HTLV-III, it was seen that the animal infused with normal human plasma (CH140) had a false positive reaction (i.e., there was no difference between the blocking with immune or nonimmune sheep antiserum) and was therefore negative for antibodies to HTLV-III. The confirmatory test showed that all of the remaining samples were specifically positive for HTLV-III antibodies which were either passively transferred or actively produced. Additional positive and negative human controls were also tested with the appropriate expected result (Table 1).

## TABLE 1

### Confirmatory Blocking of Anti-HTLV-III Sera With Sheep Anti-HTLV-III Heteroantisera

| | P/N ELISA Absorbance Ratio | |
| --- | --- | --- |
| Specimen No. | With Normal Sheep Serum | With anti-HTLV-III |
| Chimp 132 post AIDS inoculum | | |
| Passive antibody | 16.31 | 6.96 |
| Chimp 114 post AIDS inoculum | | |
| Passive antibody | 18.25 | 7.84 |
| Active antibody | > 20 | 4.51 |
| Chimp 133 post AIDS inoculum | | |
| Passive antibody | 12.34 | 5.07 |
| Active antibody | > 20 | 12.11 |
| Chimp 140 post control inoculum | | |
| Passive antibody | 9.16 | 9.16 |
| Positive Control Serum | > 20 | 9.16 |
| Positive Control Serum | 11.25 | 3.39 |
| Negative Control Serum | .90 | 1.05 |

Example 2

Comparison of sensitivies of antibody detection by indirect ELISA, Western blotting, and the competitive ELISA procedures

Two positive AIDS sera were subjected to serial 3-fold dilutions in a normal negative serum. The use of negative serum as diluent provided a constant negative background. Conditions were adjusted so that the starting concentrations of positive antibodies in each positive series were about the same. In addition, false positive sera were created by "spiking" a normal negative serum with normal negative human IgG to give IgG concentrations three times and ten times over the normal limits. The results in Table 2 indicate that using the usual criteria of a P/N >5, "Western" blotting is approximately 10-fold more sensitive than the standard indirect ELISA test, while the present test is approximately 10-fold more sensitive than the "Western" blotting procedure. The "false-positive" sera would have been scored as positive by the indirect ELISA procedure but were scored as negative by both the "Western" blotting procedure and the present competitive ELISA procedure.

## TABLE 2

### Comparison of Sensitivities of HTLV-III Antibody Detection by Indirect ELISA, competitive ELISA and Western Blot Procedure

| Sample | Titer | ELISA (P/N) | | Competitive Elisa (P/N) | | Western Blot |
|--------|-------|-------------|---|-------------------------|---|--------------|
| W8539 | 18000. | 12.80 | + | 0.79 | − | + |
| | 6000. | 11.49 | + | 0.50 | + | + |
| | 2000. | 9.64 | + | 0.22 | + | + |
| | 666. | 6.34 | + | 0.13 | + | + |
| | 222. | 4.26 | +− | 0.12 | + | + |
| | 74. | 3.28 | +− | 0.08 | + | + |
| | 25. | 1.59 | − | 0.13 | + | +− |
| | 8. | 9.93 | − | 0.26 | + | +− |
| | 2.7 | 1.21 | − | 0.49 | + | − |
| | 0.9 | 1.15 | − | 0.59 | − | − |
| | 0.3 | 1.03 | − | 1.10 | − | − |
| | 0.1 | 1.08 | − | 1.32 | − | − |
| S0137 | 18000. | 12.27 | + | 0.50 | + | + |
| | 6000. | 9.61 | + | 0.26 | + | + |
| | 2000. | 9.02 | + | 0.16 | + | + |
| | 666. | 6.46 | + | 0.09 | + | + |
| | 222. | 5.18 | + | 0.07 | + | + |
| | 74. | 3.28 | + | 0.12 | + | + |
| | 25. | 2.75 | − | 0.12 | + | +− |
| | 8. | 1.82 | − | 0.22 | + | +− |
| | 2.7 | 1.14 | − | 0.35 | + | − |
| | 0.9 | 1.19 | − | 0.78 | − | − |
| | 0.3 | 1.07 | − | 1.23 | − | − |
| | 0.1 | 0.96 | − | 1.55 | − | − |
| S1421 | false + | 12.60 | + | 1.30 | − | − |
| S1422 | false + | 13.23 | + | 1.70 | − | − |

### Example 3

Comparison of the indirect ELISA, "Western" blotting, and competitive ELISA procedures for determination of HTLV-III antibodies in normal donor and AIDS-risk donor sera

Sera were tested by indirect ELISA screening for sera with HTLV-III antibody reactivities with a P/N greater than the mean + 1.5 standard deviation (P/N greater than 3). These samples were then confirmed by the "Western" blotting procedure to identify sera reactive with verified HTLV-III protein bands. The same group of sera were retested using the inventive competi tive ELISA procedure. There was perfect concordance between the two tests for negative normal blood donors and positive asymptomatic homosexual males (AIDS-risk). In addition, 5 donors questionable by "Western" blotting were resolved into 2 positives and 3 negatives by competitive ELISA tests. Two of the 248 "Western" blot negative AIDS-risk sera were weakly positive, and 10 samples which were questionable by "Western" blot were all negative by the present competitive ELISA test. See Table 3.

## TABLE 3

### Comparison of Western Blot and Competitive ELISA Test Results: Normal Blood Donors and Asymptomatic Homosexual Males

|  |  | | ELISA | | |
|  |  |  | − | + | +/− |
| **Normal Blood Donors** |  |  |  |  |  |
| Western Blot | Negative | 352 | 352 | 0 | 0 |
|  | Positive | 0 | NA | NA | NA |
|  | ? | 5 | 3 | 2 | 0 |
| **Asymptomatic Homosexual Males** |  |  |  |  |  |
| Western Blot | Negative | 248 | 246 | 0 | 2 |
|  | Positive | 65 | 0 | 65 | 0 |
|  | ? | 10 | 10 | 0 | 0 |

## Example 4

Distribution of HTLV-I antibody among African donors

An indirect ELISA microtest to detect serum antibodies was used. Briefly, HTLV-1 was purified by rate-zonal ultracentrifugation, disrupted, and coated into the wells of microtiter plates. Portions (5 ul) of test sera, control positive sera, and control negative human sera were incubated overnight at 4°C in wells containing 100 l of 20 percent heat-inactivated normal goat serum and were quantified by measurement of absorbance at 490 nm after reaction with peroxidase-labeled goat antiserum to human IgG.

Sera with absorbance values two times greater than the normal control level were verified primarily by confirmatory neutralization which involved the same procedure as the screening test but included an additional 2-hour incubation period before the test sample was incubated with the antigen-coated wells. During this extra 2 hours of incubation, the wells were exposed to unlabeled sheep antibody to HTLV-I which reacted with and saturated HTLV antigen sites on the well, thus preventing the test serum from attaching to the well in the subsequent step. As a control in the test, adjacent wells were exposed to normal sheep serum during the additional incubation period. Sheep antiserum was used at a dilution of 1:2 and had a titer of 100,000 or more. Sheep antiserum showing reactivity with proteins from phytohemagglutinin (PHA)-stimulated human lymphocyte preparations coated on microtiter plate wells was absorbed with PBA lymphocyte preparations until the reactivity was removed. The sheep antiserum used in these experiments required absorption with one volume of cell equivalents per three volumes of serum to reach the end point. A suppression of the absorbance by 50 percent in the sample exposed to the unlabeled sheep antiserum to HTLV-I, relative to a standard normal human serum, was considered a positive confirmatory result for the presence of antibody to HTLV-I.

Sera failing the confirmatory test were absorbed with detergent-released cytosols prepared from PHA-stimulated normal human lymphocytes and with HTLV-I-producing cells and retested for binding to HTLV-I. Samples were scored positive if the difference between absorption with virus-positive and virus-negative cell preparations was greater than 50 percent. Titers of positive sera were determined by serial dilution, regression analysis of the titration curves, and solving for the dilution, giving results equivalent to a 1:20 dilution of the reference negative control serum tested in wells of the same plate. In Table 4, R is the ratio of the sample to the negative control; all groups followed log-normal distributions of R.

TABLE 4

Distribution of HTLV-I Antibody Among African Donors

| Geographic and Racial Background of Donors | Group Characteristics | Number Tested | R Median | Number With R $\geq$ 2 | Number Positive |
|---|---|---|---|---|---|
| Egypt, white | Infectious disease clinic (no malignancies) | 101 | 0.90 | 12 | 2 |
| Tunisia, white | Malignant lymphoma | 22 | 1.31 | 7 | 2 |
| | Mammary carcinoma | 256 | 2.10 | 136 | 6 |
| Ghana, black | Burkitt's patients | 610 | 3.32 | 336 | 52 |
| | Normal comparison population | 236 | 3.30 | 200 | 19 |
| Uganda, black | Burkitt's patients and normal comparison population | 86 | 1.71 | 31 | 18 |
| Nigeria, black | T-cell lymphoma | 9 | 1.60 | 2 | 2 |
| South Africa | | | | | |
| Cape Town, black and white | All donors | 283 | 0.90 | 38 | 15 |
| | Lymphoid malignancy | 22 | 0.86 | 2 | 1 |
| | Myeloid malignancy | 104 | 0.84 | 16 | 9 |
| | Solid tumors | 59 | 1.02 | 11 | 3 |
| | Nonmalignant disease and healthy blood donors | 98 | 0.80 | 9 | 2 |
| Johannesburg, black | Healthy blood donors | 104 | 0.9 | 5 | 0 |

0 234 941

Example 5

HTLV-III was purified, disrupted, and coated into the wells of microtiter plates. The purification process included treating the HTLV-III antigen with guanidine hydrochloride (4 to 8 molar), thus destroying certain cellular antigens while preserving the immunoreactivity of the HTLV antigens.

Reaction buffers were then added to the wells of the microtiter plates. These buffers contain 4/8 mixture of sheep plasma (either normal or immune); 3/8 solution of 20% fetal bovine serum, and phosphate buffered saline; and 1/8 lymphocyte extract. To prepare the lymphocyte extract (or uninfected cells), a detergent extract of leucocyte is made of 1 gram of fresh lymphocyte suspended in three volumes of 5% NP40, 50 millimolar Tris (pH 7.4) and 1 millimolar PMSF. The extract also contains Trasylol, 10 millimolar magnesium chloride, and .25 molar glycerol. This mixture was disrupted by vortexing or homogenation with a loose fitting homogenizer, and the nuclei removed by centrifugation; the remaining supernatant was the lymphocyte extract. The microtiter plates containing the purified antigen were incubated with the above-described buffer for two hours, thus exposing antigens on the plate to unlabeled sheep antibody which reacted with and saturated HTLV-III antigen sites on the well, thus preventing the test serum from attaching to the well in the subsequent step.

Portions (5ul) of test sera, control positive sera, and control negative sera were incubated overnight at 4°C in wells containing 100ul of 20% heat-inactivated normal goat serum. The test sera was then incubated with the antigen coated wells. The suppression of absorbance by more than 50% in the sample exposed to the unlabeled sheep antiserum to HTLV-III, relative to a standard normal human serum, was considered a positive confirmatory result for the presence of antibody to HTLV-III.

The above-noted competitive ELISA process was particularly useful in the detection of HTLV in problem sera--sera which scores positive in the ELISA system presently available commercially, but scores false on the competitive ELISA of the present invention and other competitive ELISA systems. These problem sera include HLA (particularly in multiparous women) and in sera exhibiting anti-mitochondrial activity.

The quantities of sheep plasma, goat serum, fetal bovine serum and, lymphocyte extract may be varied by at least ± 10%, where expressed as a percentage and ± 1/8 where expressed as a fraction of 8 to remain within the scope of the present invention.

**Claims**

1. An enzyme linked immunosorbent assay for detection of human T-cell lymphotropic virus in a sample, characterized in the use of a reaction buffer containing about 4/8 sheep plasma, about 3/8 solution of 20% goat serum, 20% fetal bovine serum, and phosphate buffered saline, and about 1/8 lymphocyte extract; and further characterized by treating a target antigen used in the enzyme linked immunosorbent assay with a guanidine salt.

2. The assay of Claim 1, further characterized in that said lymphocyte extract is a leucocyte extract comprising fresh lymphocytes suspended in a nonionic detergent, 2-amino-2-hydroxymethyl-1,3-propanediol, phenyl methyl sulfonyl fluoride, magnesium chloride and glycerol.

3. An improved enzyme linked immunosorbent assay for detection of human T-cell lymphotropic virus in a sample, characterized in that it includess:

treating a human T-cell lymphotropic virus target antigen with a guanidine salt having a soluble counter ion, and then

treating said target antigen with a solution of sheep plasma, hetero animal antiserum and a lymphocyte extract.

4. The assay of Claim 3, furhter characterized in that said lymphocyte extract is a leucocyte extract comprising fresh lymphocytes suspended in a nonionic detergent, 2-amino-2-hydroxymethyl-1,3-propanediol, phenyl methyl sulfonyl fluoride, magnesium chloride and glycerol.

5. The assay of Claim 3, further characterized in that said hetero animal antiserum is 20% normal goat serum, 20% fetal bovine serum, and phosphate buffered saline.

6. The assay of Claim 3, further characterized in that said guanidine salt is guanidine hydrochloride.

7. An assay method for detecting human T-cell lymphotropic virus type III in a sample, characterized in that a test antigen is treated with a guanidine salt selected from the group consisting of guanidine hydrohalide, guanidine nitrate, and guanidine thiocyanate.

8. An assay method for detecting human T-cell lymphotropic virus or the like, as claimed in any one of claims 1-6, characterised in that the human T-cell lymphotropic virus target antigen is derived from human T-cell lymphotropic virus type III, or a similar virus, being a causative agent of acquired immune deficiency syndrome (AIDS).